Europäisches Patentamt

European Patent Office

Office européen des brevets

(11) Veröffentlichungsnummer : **0 096 191**
**B1**

(12)

# EUROPÄISCHE PATENTSCHRIFT

(45) Veröffentlichungstag der Patentschrift :
**30.12.86**

(21) Anmeldenummer : **83103620.7**

(22) Anmeldetag : **14.04.83**

(51) Int. Cl.⁴ : **A 61 M 5/00**, **A 61 J 1/00**,
**B 65 D 30/08**

(54) Beutel für Infusionslösungen oder dergleichen und Verfahren zu dessen Herstellung.

(30) Priorität : **15.05.82 DE 3218415**

(43) Veröffentlichungstag der Anmeldung :
**21.12.83 Patentblatt 83/51**

(45) Bekanntmachung des Hinweises auf die Patenterteilung : **30.12.86 Patentblatt 86/52**

(84) Benannte Vertragsstaaten :
**AT BE CH DE FR GB IT LI LU NL SE**

(56) Entgegenhaltungen :
**EP-A- 0 015 775**
**AT-A- 360 138**
**DE-A- 2 908 117**
**DE-A- 3 038 971**
**GB-A- 911 143**
**GB-A- 2 068 333**

(73) Patentinhaber : **Sengewald, Karl-Heinz, Dr.**

**D-4802 Halle in Westfalen (DE)**

(72) Erfinder : **Sengewald, Karl-Heinz, Dr.**

**D-4802 Halle in Westfalen (DE)**

(74) Vertreter : **Baur, Eduard, Dr.-Ing. Dipl.-Ing. et al**
**Werderstrasse 3**
**D-5000 Köln 1 (DE)**

**Beschreibung**

Die Erfindung betrifft einen Beutel für Infusionslösungen oder dergleichen und Verfahren zu deren Herstellung und Befüllen, beispielsweise (CAPD-Lösungen) für eine kontinuierliche ambulante Peritonealdialyse, aber auch Infusionslösungen. Diese müssen aus sterilen Behältnissen zugegeben werden. Vielfach gebräuchlich sind Flaschen aus Glas oder Kunststoff. Da diese nach dem Blasverfahren hergestellt werden, wäre es nur unter größten Schwierigkeiten möglich, die Luft zum Aufblasen der Flaschen keimfrei zu gestalten. So müssen die Flaschen vor ihrer Befüllung sorgfältig gereinigt und sterilisiert werden.

Durch die GB-A-911 143 ist ein Verfahren zum Herstellen von Beuteln in der Weise bekannt, daß aus einem unter aseptischen Bedingungen hergestellten Schlauch durch quer zur Schlauchlängsachse verlaufende Trenn- und Schweißschnitte die einzelnen Beutel hergestellt werden. Um die Beutel bei kranken Personen mit Sekreten zu befüllen, wird eine Ecke abgeschnitten und ein Röhrchen eingesteckt. Die aus einer Folienlage bestehenden Beutel eignen sich nicht zum Befüllen mit Infusionslösungen, da es notwendig ist, die durch Abschneiden einer Beutelecke gebildete Einfüllöffnung zu öffnen und nach der Befüllung durch Schweißung der Folienlagen zu schließen.

Zum Aufbewahren von Nahrungsmitteln wird in der EP-A-00 15 775 vorgeschlagen, einen Beutel aus zwei verschiedenen Folienlagen herzustellen. So geht ein Vorschlag dahin, die Innenlage aus Polyäthylen und die Außenlage aus einem Polyamid herzustellen.

Durch die GB-A-2 068 333 ist es bekannt, zum Aufbewahren von Flüssigkeiten, die gegen Sauerstoff empfindlich sind, wie beispielsweise Wein, einen aus drei Lagen bestehenden Beutel zu verwenden, wobei die drei Folienlagen aus unterschiedlichen Kunststoffen nach dem Coextrusionsverfahren hergestellt werden. Bei diesem Coextrusionsverfahren ist es schwierig, die Lagen aus den unterschiedlichen Kunststoffmaterialien gegen andere Kunststoffmaterialien auszutauschen. Zum Herstellen des Beutels wird ein Folienabschnitt in der Mitte umgeschlagen und an zwei sich kreuzenden Rändern verschweißt, während der verbleibende Rand die Einfüllöffnung darstellt, die sich somit über die Breite des Beutels erstreckt. Da zur Herstellung des Beutels ein flacher Folienschnitt gefaltet wird, wird der Beutel nicht unter aseptischen Bedingungen hergestellt. Auch ist seine sich über die Breite erstreckende Einfüllöffnung nicht zum Befüllen mit einer Infusionslösung unter aseptischen Bedingungen geeignet. Der Beutel ist entsprechend zum Aufbewahren von Wein vorgeschlagen worden.

Aus der DE-A-30 38 971 ist ein Beutel für medizinische Zwecke bekannt, dessen beide Wandungen aus zwei oder sogar drei Folienlagen bestehen. Zur Herstellung der Beutel werden die erhaltenen Folienlagen so aufeinander gelegt, daß ihre Oberflächen einander gegenüberliegen, d.h. die beiden Folienlagen, die ursprünglich aus einem Schlauch geschnitten worden sind, werden gewendet. Beim Wenden der Folien können sich Verunreinigungen anlagern.

Aus der Praxis ist bekannt, in diesem Zusammenhang kann auch hingewiesen werden auf die AT-A-360 138, für Infusionslösungen Beutel aus thermoplastischer Kunststoffolie in Gestalt eines Weich-PVC (Polyvinylclorid) zu verwenden. Zur Herstellung wird ausgegangen von einem unter aseptischen Bedingungen geblasenen Schlauch, wobei bei flachliegendem Schlauch dessen Breite der Breite des herzustellenden Beutels entspricht, d. h. daß keine Schweißung der Folienlagen an den in Schlauchrichtung verlaufenden beiden Längskanten vorhanden ist. Sofern somit Beutel unterschiedlicher Breite oder bei gefülltem Beutel unterschiedlichen Durchmessers erhalten werden sollen, ist es notwendig, von einem Schlauch entsprechender Breite auszugehen, so daß zur Herstellung dieser Infusionsbeutel mit unterschiedlichen Breiten oder Durchmessern eine entsprechende Vielzahl von Blasformen vorhanden sein muß. Der Bodenbereich des Beutels ist mit im Abstand zueinander befindlichen Querabschweissungen versehen, zwischen denen eine Stanzöffnung zum Aufhängen des Beutels bei seiner Anwendung vorhanden ist.

Bei dem vorbekannten, aus Weich-PVC bestehenden Infusionsbeutel sind zum Befüllen und auch zur Entnahme besondere Anschlußteile vorhanden, die dadurch gebildet sind, daß zwischen den beiden Folienlagen des Schlauches gesonderte und mit dem Schlauchmaterial anschließend verbundene Röhrchen aus Kunststoff angeordnet sind. Auch diese besondere Anordnung der Röhrchen verteuert die Herstellung des Infusionsbeutels.

Diese Röhrchen haben, solange deren vorderen freien Ende nicht verschlossen sind, dies geschieht in der Regel erst, nachdem die Beutel mit der Infusionslösung befüllt worden sind, den Nachteil, daß sich an den von außen frei zugänglichen Innenwandungen der Röhrchen schädliche Partikel, so beispielsweise aus der Luft, anlagern können. Auch kann diese Luft noch in einem gewissen Bereich zwischen die beiden Folienlagen des Beutels gelangen, da an den vorderen, in den Beutel hineinragenden Enden der offenen Röhrchen, weil deren Wandungen nicht einander anliegen, auch die beiden Folienlagen, die den Schlauch des Beutels bilden, nicht anliegen.

Es ergibt sich somit, daß die Beutel aus Weich-PVC mit eingesetzten Röhrchen vielfach nicht keimfrei zu halten sind und vor ihrer Befüllung im Bereich der Röhrchen sterilisiert werden müßten. Sofern auch die Herstellung des Ausgangsschlauches unter aseptischen Bedingungen erfolgt sein sollte, so ist bei den bekannten, aus Weich-PVC bestehenden Beuteln mit den Einfüll- oder Entleerungsröhrchen vor seiner Befüllung eine weitere Sterilisation notwendig. Die in der vorerwähnten AT-A 360-138 dargestellte und beschriebene Anordnung und Absicherung der Einfüll- und Entnahmeöffnung ist sehr schwierig herstellbar und in der Praxis nicht verwendet worden.

Infusionsbeutel aus Weich-PVC lassen sich sehr leicht verschweißen, haben aber keine große Festigkeit.

Bisherige Beutel mit Infusionslösungen werden nach der Füllung einer Sterilisation unterworfen. Bei dieser Sterilisation werden vorhandene schädliche Partikel abgetötet. Dabei bleiben diese aber im Beutel vorhanden.

Die vorliegende Erfindung geht von der Aufgabe aus, einen Beutel für Infusionslösungen zu schaffen, der sich einfach und unter aseptischen Bedingungen dauerhaft steril herstellen und befüllen läßt sowie sich bei hoher Festigkeit für verschiedenartigste Infusionslösungen eignet.

Zur Lösung dieser Aufgabe wird bei einem Beutel für Infusionslösungen, oder dergleichen, der ausgehend von einem unter asepetischen Bedingungen hergestellten Schlauch aus thermoplastischer Kunststoffolie durch Verschweißung der beiden Schlauchwandungen und Trennschnitte durch die Schlauchwandungen hergestellt und mit einem nach außen vorstehenden, durch Stopfen verschließbaren Anschlußteil oder mehreren solcher Anschlußteile versehen ist, erfindungsgemäß vorgeschlagen, daß die beiden gegenüberliegenden Schlauchwandungen jeweils aus einer Verbundfolie bestehen, die Verbundfolien durch die Umfangskontur des Beutels nachzeichnenden Schweißungen miteinander verbunden und außen neben diesen Schweißungen die Trennschnitte vorhanden sind sowie das Anschlußteil oder die Anschlußteile mit dem Beutel einstückig ist bzw. sind.

Der erfindungsgemäße Beutel für Infusionslösungen hat durch die Maßgabe, daß er aus einer Verbundfolie hergestellt ist, zunächst eine große Festigkeit und Beständigkeit, weil die miteinander verbundenen Folienlagen auch nach den Gesichtspunkten ihrer Festigkeit und ihrer leichten Verbindung durch Schweißung sowie der Beständigkeit gewählt werden können.

Durch den weiteren Vorschlag, daß die Verbundfolien umlaufend durch Schweißungen miteinander verbunden und neben diesen Schweißungen die Trennschnitte vorhanden sind, wird erreicht, daß die Beutel ohne eine Öffnung des unter aseptischen Bedingungen bei einer Extrusionstemperatur von ca. 170 °C hergestellten Innenschlauches gefertigt werden und nach der Fertigstellung entsprechend geschlossen bleiben und dann die Trennschnitte angebracht werden. Die umlaufende Verschweißung besagt weiterhin, daß in einer breiten Schlauchbahn nebeneinander gleichzeitig zwei Beutel herstellbar sind. Dieser Vorschlag besagt auch, daß die äußeren Folienlagen beidseitig des Schlauches nicht den Innenschlauch umgreifen müssen oder bis zu den Schlauchkanten reichen müßten. Dadurch ist eine vereinfachte Herstellung des mehrlagigen Schlauches möglich.

Durch die Maßgabe, daß das Anschlußteil oder die Anschlußteile mit dem Beutel einstückig sind und somit ebenfalls aus den vorgenannten Verbundfolien bestehen, ist der Vorteil vorhanden, daß in gleicher Weise, wie die Folienlagen des Beutels dicht aneinander anliegen, auch die Folienlagen der Anschlußteile dicht einander anliegen, so daß in die Anschlußteile bzw. in den Beutel keine Bakterien oder dergleichen schädliche Stoffe gelangen können.

In weiterer erfindungsgemäßer Ausgestaltung wird vorgeschlagen, daß das Anschlußteil oder die Anschlußteile zur Schlauchlängskante einen Abstand haben. Dadurch ist ein einfaches Öffnen des Anschlußteiles oder der Anschlußteile durch Aufschneiden der Schlauchlängskante möglich. Da im Bereich der Schlauchlängskante die Folien nicht einander anliegen, ist dort das Abheben der Folienlagen zum Zwecke der Befüllung vereinfacht. Auch bleiben durch diesen Vorschlag die Anschlußteile bis zum Aufschneiden der Schlauchlängskante in der Abfüllstation geschlossen, so daß das Befüllen der Beutel in der Abfüllstation unter aseptischen Bedingungen stattfinden kann. Dieses Befüllen wird vereinfacht durch den weiteren Vorschlag, daß die vorderen Enden der Anschlußstücke zur Längskante des Schlauches einen Abstand haben. So kann der von den Anschlußteilen freie Schlauchlängskantenbereich mit dem Transport des Schlauches dienenden Elementen versehen sein, so in Gestalt von Ausschnitten, wie sie beispielsweise bei Filmen zum Herstellen von Fotografien vorhanden sind. Der Bereich des Schlauches zwischen einer längsverlaufenden Kante und den Anschlußteilen kann aber auch ohne die besondere, dem Transport des Schlauches dienenden Elemente versehen sein mit der Maßgabe, daß die Transporteinrichtungen auf diesen Bereich einwirken und dabei diesen sogar beschädigen können. So ist es möglich, daß der Schlauch zu seinem Transport in die Abfüllstation oder zur Verpackungseinrichtung von Transportelementen in diesem Bereich erfaßt wird, beispielsweise durch zu beiden Seiten anliegenden Räder, insbesondere als Reibräder mit rauher Oberfläche. Auch können in diesem Bereich zwischen Schlauchlängskante und Anschlußteilen an beiden Seiten des Schlauches umlaufende Riemen anliegen, auch mit einem solchen Anpreßdruck, daß dieser Schlauchbereich in gewisser Weise beschädigt werden kann, weil dieser Bereich später ohnehin Abfallprodukt sein wird.

Durch diese Maßgabe wird auch erreicht, daß die Anschlußteile zum Befüllen des Beutels, aber auch, um diese beispielsweise mit Stopfen oder dergleichen zu versehen, in einfacher Weise beispielsweise von der Seite her zugänglich sind.

Die Maßgabe, daß die Anschlußteile zu der Schlauchlängskante einen Abstand haben, dient ebenfalls zur Verwirklichung der wichtigen Maßgabe, daß die auf den inneren Schlauch aufgebrachten Folienlagen nicht den inneren Schlauch vollkommen umschließen müssen, sondern zu den beiden Schlauchlängskanten einen Abstand haben können.

Die Lösung, die vorderen Enden der Anschlußteile des Beutels mit einem gewissen Abstand zur Schlauchlängskante anzubringen, hat den weiteren wesentlichen Vorteil, daß eine weitere Sicherung der « Öffnungen » der Anschlußteile beim Transport oder Lagerung des Schlauches mit der Beutelkette gegeben ist. Nach einem weiteren Merkmal der Erfindung ist dieser Bereich ein weiteres Sicherungselement, das die Eingänge der Anschlußteile schützt und auch nach außen abschließt, sofern die vorderen Öffnungen der Anschlußteile nicht durch Haftung der Innenlagen der Folien oder Anklebung der Innenlagen der Folien ausreichend verschlossen sein sollten. Der streifenförmige Bereich zwischen dem vorderen Ende der Anschlußteile und der Schlauchlängskante bildet nämlich eine Sperrzone, weil auch in diesem Bereich die Schlauchwandungen einander anliegen, insbesondere, sofern keine dem Transport dienende Ausnehmungen vorhanden sind.

In weiterer erfindungsgemäßer Ausgestaltung sind die nach außen vorstehenden Anschlußteile konisch und bilden Kanäle, deren Querschnitte sich in Richtung von außen nach innen verjüngen. Dadurch ist es möglich, in diese Anschlußstücke weitere Anschlußelemente einfach und mit sicherer Haftung anzubringen.

Ein weiterer Vorschlag geht dahin, daß der Beutel in seinem Bodenbereich und in seinem Kopfbereich mit zwei im Abstand zueinander befindlichen Querabschweißungen versehen ist.

Da die erfindungsgemäßen Beutel ausgehend von einem sterilen Schlauch hergestellt sind und die Beutel bis zu ihrer und während ihrer Befüllung steril bleiben, ist es möglich, die Beutel nach ihrer Herstellung im Schlauch oder am Schlauch zu belassen und den Schlauch nicht unmittelbar zu verarbeiten, sondern zu einer Rolle aufzuwickeln und an einem anderen Ort zu befüllen, ohne daß durch eine längerfristige Lagerung oder den Transport das Innere der Beutel kontaminiert werden könnte.

Der Schlauch mit darin oder daran angebrachten Beuteln kann somit selbständige Handelsware sein. Es kann auch dargelegt werden, daß die Anschlußteile mit ihren Öffnungen in einer Reihe hintereinander angeordnet sind. Da sie von Beutel zu Beutel einen gleichbleibenden Abstand haben, kann mit dieser Anordnung ein maschinelles Einsetzen von Stopfen, Einfülleitungen oder dergleichen erfolgen. Diese maschinelle vollautomatische Arbeitsweise des Einsetzens von Stopfen oder das Einführen von Füllleitungen, gegebenenfalls auch in unmittelbarem Anschluß an die Befüllung das Schließen der Einfüllöffnung, ist besonders vorteilhaft in Verbindung mit der Maßgabe, daß während der vorbeschriebenen Arbeiten die Beutel im oder am Schlauch bleiben und durch den Schlauch zu einer Beutelkette verbunden sind. Dies wird erreicht durch die Maßgabe, daß zwischen den einzelnen, durch Verschweißung der Folienlagen des Schlauches gebildeten Beuteln durch beide Schlauchwandungen durchgehende Perforationsschnitte angeordnet sind. Die Perforationsschnitte haben jeweils eine solche Länge mit dazwischen verbleibenden Stegen, daß eine ausreichende Festigkeit vorhanden ist, um den Schlauch, nachdem in diesen die Beutelkonturen angebracht worden sind, zu einer Rolle aufzuwickeln oder auch den Schlauch mit den Beuteln durch die Befülleinrichtung als Beutelkette transportieren zu können.

Es wurde bereits dargelegt, daß, da die Beutel durch Verschweißung der beiden Folienlagen des Schlauches hergestellt worden sind, der Schlauch geöffnet werden kann, auch sofern die Anschlußteile Öffnungen haben, die nicht durch eine besondere Verschweißung geschlossen sind.

Es ist zu berücksichtigen, daß die beiden Folienlagen, die den Schlauch oder Beutel bilden, hierunter sind auch die Verbundfolien zu verstehen, mit einer gewissen eigenen Adhäsion aneinander anliegen. Um diese zu erhöhen, kann bei der Herstellung des Schlauches vorgesehen sein, daß die einander anliegenden Innenlagen des Schlauches aus Verbundfolie in gewisser Weise leicht ankleben. Dies kann beispielsweise dadurch geschehen, daß in einem gewissen zeitlichen oder räumlichen Abstand nach dem Blasen des Ausgangsschlauches dessen Innenwandungen angedrückt werden zu einem Zeitpunkt, bei dem die Folienlagen durch die noch vorhandene Erhitzung leicht aneinander ankleben. Diese geringe Verklebung kann dabei nicht lediglich durch die Temperatur des Folienschlauches zum Zeitpunkt der Aneinanderbringung geregelt werden, sondern auch durch Walzen, die mit einem entsprechend regulierbaren Druck die beiden Folienlagen des Schlauches aneinanderlegen. Diese geringe Klebkraft ist so bemessen, daß keine Luft durch die Einfüllöffnung gelangen kann.

Es sei verstanden, daß die vorbeschriebene Maßnahme, die inneren Folienlagen in eine leichte Klebung zu bringen, bevorzugt nur im Bereich der einstückigen Anschlußteile vorgenommen wird. Dies bedeutet, daß beispielsweise in einem kurzen Zeitraum nach der Herstellung des Innenschlauches von beiden Seiten des Schlauches zwei Walzen geringer Länge vorhanden sind, die nur in einem örtlichen Bereich der nachfolgend anzubringenden Anschlußteile der Beutel wirksam sind. Die geringe Anklebung der inneren Folienlagen aneinander im Bereich der Anschlußteile ist möglich, weil dort später ohnehin durch Einführen eines Stöpsels oder Einfülleitung ein mechanisches Auseinandertreiben der beiden Folienlagen erfolgt. Aus dieser Schilderung ist zu ersehen, daß im Bereich der Anschlußteile, möglicherweise auch nur an deren vorderen Enden, auch eine gewisse stärkere Klebung unter Nutzung der Extrusionshitze erfolgen kann, weil die Folienlagen durch mechanische Krafteinwirkung einander abgehoben werden.

Die Erhaltung der Sterilisation der Beutel unabhängig davon, ob die Innenwandungen der Anschlußteile durch Adhäsion oder geringe Anklebung einander anliegen, kann dadurch erhöht werden, daß trotz Anbringung der Perforationsschnitte der Schlauch nicht geöffnet wird. Dies geschieht in erfindungsgemäßer Ausgestaltung durch die Maßgabe, daß die Perforationsschnitte durch erhitzte, mit Zacken versehene Messer erhalten werden und dabei die Schnittflächen der Folien durch Schweißung

4

miteinander verbunden sind. Durch diese Maßnahme wird somit selbst durch die Anbringung der Schnitte außen an die Schweißungen zur Bildung der Beutel der Schlauch nicht geöffnet, weil an den Schnittflächen mit deren Anbringung durch die Verschweißung der Folienlagen ein sofortiger Verschluß durchgeführt wird.

Besonders vorteilhaft besteht die Innenlage aus einem Polyäthylen (PE), und zwar in weiterer Ausgestaltung aus einem Polyäthylen mit angehobener Dichte, die vorzugsweise 0,935 bis 0,960 g·pro cm³ beträgt und einer Dicke von 75 μm. Sehr gut geeignet ist auch Polypropylen.

Die Außenlage besteht aus einer Folienlage aus vorzugsweise Polyamid. Die Herstellung der Beutel für Infusionslösungen aus einer Verbundfolie macht es möglich, daß als Innenlage eine Folie gewählt wird, die neben der guten Schweißbarkeit aus einer Zusammensetzung besteht, die den verschiedenartigsten Infusionslösungen gegenüber neutral ist. Hierzu ist besonders ein Niederdruck-Polyäthylen (ND-PE) geeignet.

Die Außenlage wird gewählt nach dem Gesichtspunkt der Dichtigkeit gegen Gas- und Wasserdampf und auch dem Schutz vor den Einwirkungen einer Strahlung, beispielsweise Sonnenbestrahlung, auf die Infusionslösung. Zugleich soll sie einfach bedruckbar sein. Eine Folie, die dazu besonders geeignet ist, weil sie eine Vielzahl von Vorteilen in sich vereinigt, ist eine ungereckte Polyamidfolie (Polyamid 6).

In weiterer erfindungsgemäßer Ausgestaltung wird vorgeschlagen, daß jede Wandung des Beutels aus mehr als zwei Folienlagen besteht, so beispielsweise in Richtung von außen nach innen aus Polyamid (PA) Polyäthylen (PE), Polyvinylidenchlorid (PVDC) und Polyäthylen (PE). In dieser, aus vier Folienlagen bestehenden Wandung kann auch in Richtung von außen nach innen die zweite Lage aus Polyäthylen (PE) wegfallen.

Besonders vorteilhaft ist auch folgende aus drei Schichten bestehende Wandung Polyamid (PA), orientiertes Polyester mit X-Beschichtung (OPX) und Polyäthylen (PE).

Polyvinylidenchlorid hat eine sehr hohe Dichtigkeit gegen die Einwirkung von Sauerstoff, so daß insbesondere Lösungen mit oder aus Aminosäure geschützt werden.

Die Herstellung des aus mehreren Lagen bestehenden Schlauches kann nach dem Coextrusionsverfahren erfolgen. Besonders vorteilhaft ist jedoch das erfindungsgemäße Verfahren, daß zunächst unter aseptischen Bedingungen ein Schlauch aus thermoplastischer Kunststoffolie, insbesondere aus Polyäthylen, hergestellt und an diesen Schlauch zu beiden Seiten jeweils eine Folienlage aus anderem thermoplastischen Kunststoff, insbesondere aus einem Polyamid, durch Kaschierung angebracht und aus diesem Schlauch die Beutel in der Weise hergestellt werden, daß die so erhaltenen beiden Verbundfolien der Umfangskontur der Beutel entsprechend durch Schweissungen miteinander verbunden werden und außen neben diesen Schweissungen Trennschnitte angebracht werden. Durch diesen Vorschlag ist es möglich, an den Schlauch in einfacher Weise durch einen Kaschierkleber eine Folie besonderer Wahl anzubringen. Auch kann der Hersteller der Beutel mit einem aufgewickelten Schlauch beliefert werden, der dann durch Kaschierklebung eine oder mehrere weitere Folienlagen aufbringt.

In weiterer erfindungsgemäßer Ausgestaltung wird vorgeschlagen, daß der Schlauch aus thermoplastischer Kunststoffolie unter aseptischen Bedingungen bei einer Extrusionstemperatur von ca. 170 °C hergestellt wird und an dem hermetisch geschlossenen Schlauch die Verschweißung der beiden Schlauchwandungen zur Herstellung der Beutel vorgenommen und außen neben den Schweißnähten der Beutel besäumt wird.

Ein weiterer erfindungsgemäßer Vorschlag geht dahin, daß in dem Schlauch in dichtem Abstand hintereinander und quer zur Längserstreckung des Schlauches mit zu einer Schlauchlängskante gerichteten Anschlußteilen und in einem Abstand zur Schlauchlängskante durch Verschweißung der beiden Folienlagen die Beutel hergestellt und zwischen den einzelnen Beuteln in einer Erstreckung quer zur Beutellängsrichtung durch beide Schlauchwandungen durchgehend Perforationsschnitte angebracht werden und in einer unter aseptischen Bedingungen arbeitenden Füllstation der Schlauch an seiner Kante aufgeschnitten und dadurch die Anschlußteile freigelegt und die Beutel befüllt sowie anschließend die Öffnungen der Anschlußteile geschlossen werden. Dadurch werden dann erstmalig die Einfüllöffnungen bzw. allgemeinen Öffnungen der Anschlußteile freigelegt, so daß sie, sofern sie durch Adhäsion oder geringer Klebung einander anhaften, durch Stopfen, Einfüllröhrchen oder dergleichen geöffnet werden können. Durch das Aufschneiden der Schlauchlängskante wird zugleich ein längsverlaufender Kanal gebildet, der ebenfalls zur Führung und Ausrichtung der Beutelkette an den Einfüllwerkzeugen oder Verschlußwerkzeugen dient.

Obwohl die Schlauchlängskante aufgeschnitten ist, dienen die streifenförmigen Bereiche zwischen Kante und Anschlußteilen der Beutel weiterhin dazu, die Beutelkette zu transportieren, gegebenenfalls zu einer Verpackungseinrichtung, in der die befüllten Beutel in der Perforationslinie durch Abreißen der Stege zwischen den Perforationen getrennt werden. Es ist auch möglich, daß mit dem Befüllen der Beutel die in den Perforationsschnitten vorhandenen Stege aufreißen und somit eine Trennung der Beutel vom Schlauch stattfindet.

Die Erfindung ist in den Zeichnungen beispielhaft dargestellt.

Es zeigen :

Figur 1  Beutel für Infusionslösungen in Draufsicht,

Figur 2  einen Schnitt durch einen Schlauch,

Figur 3  einen Schnitt durch einen weiteren Schlauch,

Figur 4  einen Schnitt durch einen Schlauch mit drei Folienlagen einer Wandung,

Figur 5  einen Schnitt durch einen Schlauch mit vier Folienlagen einer Wandung,

Figur 6  in perspektivischer und im wesentlichen schematischer Darstellung das Blasen des Schlauches und Herstellen der Beutel,

Figur 7  im wesentlichen schematisch und in perspektivischer Darstellung das Herstellen eines Schlauches,

Figur 8  in perspektivischer und im wesentlichen schematischer Darstellung die Anbringung von Beuteln im Schlauch zu dessen Längserstreckung,

Figur 9  in perspektivischer Darstellung das Versehen der Anschlußteile der Beutel mit Stopfen,

Figur 10  einen Teilausschnitt aus dem Folienschlauch,

Figur 11  in perspektivischer und im wesentlichen schematischer Darstellung den Transport und das Befüllen der Beutelkette.

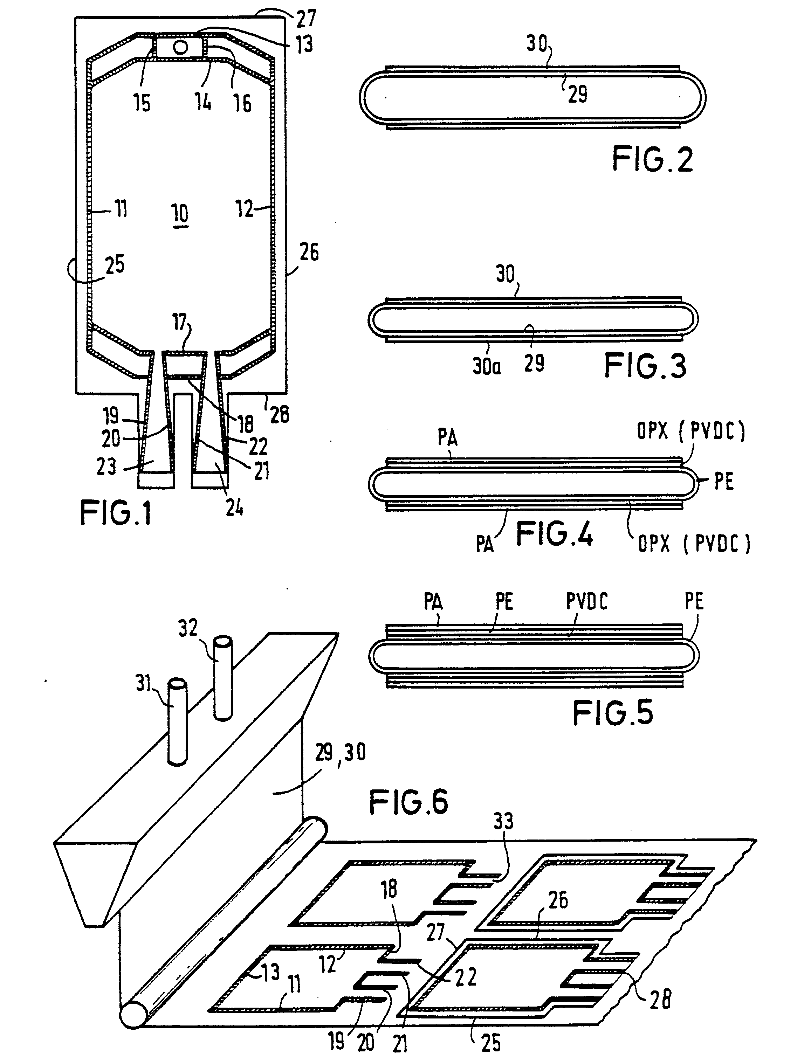

Figur 1 zeigt beispielhaft einen Beutel 10 für Flüssigkeiten, die in der Medizin angewendet werden, beispielsweise Infusionslösungen, der in Längsrichtung verlaufende Schweißnähte 11 und 12 zur Verbindung der beiden Folienlagen des Schlauches sowie an seinem Bodenende zwei in einem Abstand vorhandene querverlaufende Schweißnähte 13 und 14 hat, die durch stegartige Schweißnähte 15 und 16 miteinander verbunden sind. An seinem Kopfende hat er die querverlaufenden, ebenfalls in einem Abstand zueinander befindlichen Schweißnähte 17 und 18, die unterbrochen sind durch die Schweißnähte 19 und 20 bzw. 21 und 22, die in Längsrichtung des Beutels verlaufen und die Kanäle zum Befüllen oder Entleeren des Beutels darstellen. Die Schweißnähte 19 und 20 bzw. 21 und 22 verlaufen in Richtung zur Beutelmitte hin konvergierend, so daß sich die davon begrenzten Kanäle 23 und 24 in Richtung von außen nach innen verjüngen. In den Bereichen außerhalb der vorgenannten, die Kontur bzw. das Füllvermögen des Beutels bestimmenden Schweißnähte sind Schnitte 25 und 26 an den Längsseiten sowie 27 am Boden und 28 am Kopfende vorhanden, wobei das Kopfende im Bereich der Anschlußteile einen Schnitt 28 hat, der um die Anschlußteile in Gestalt der Kanäle 23 und 24 geführt ist. Sie sind mit dem Beutel einstückig, weil sie aus den gleichen Folienlagen gebildet sind.

Figur 2 zeigt den Ausgangsschlauch zum Herstellen von Beuteln nach Figur 1. Er ist nach dem Coextrusionsverfahren hergestellt in der Weise, daß eine innere Folienlage 29 aus einem Polyäthylen, besonders vorteilhaft aus einem Niederdruckpolyäthylen mit einer Dichte von 0,935 g pro cm$^3$ und einer Dicke von 75 μm vorhanden ist, während die Außenlage 30 aus einem Polyamid mit einer Dicke von 70 μm besteht.

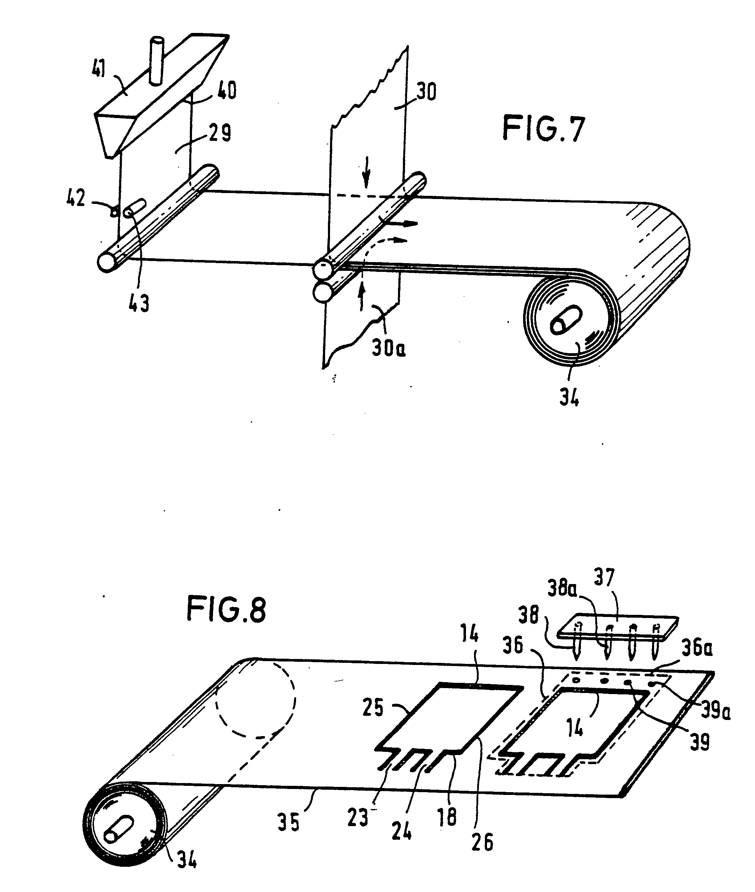

Figur 3 zeigt einen Innenschlauch 29 aus Polyäthylen, der außen mit je einer Folienlage 30, 30a durch Kaschierung belegt ist. Dessen Herstellung ist anhand von Figur 7 dargestellt.

Figur 4 zeigt einen Schlauch aus Polyäthylen (PE), an dem durch Kaschierung bzw. Aufklebung angebracht ist eine Folie aus orientiertem Polyester mit X-Beschichtung (OPX) oder Polyvinylidenchlorid (PVDC), die außen mit einer Folie aus Polyamid (PA) belegt ist.

Der Schlauch nach Figur 5 hat in Richtung von außen nach innen die Folienlagen PA, PE, PVDC und PE.

Figur 6 zeigt beispielhaft die Herstellung von Beuteln aus einer Verbundfolie. In der beispielsweise nach dem Coextrusionsverfahren arbeitenden Anlage zum Herstellen eines Schlauches werden durch die Einfüllöffnung 31 das Granulat für die Innenlage 29 aus Polyäthylen und durch die Einfüllöffnung 32 das Granulat für die Außenlage 30 aus Polyamid eingegeben. Diese Blasanlage ist hermetisch abgeschlossen, damit das Blasen des Schlauches, auch bei einer Extrusionstemperatur von ca. 170 °C, unter aseptischen Bedingungen erfolgt.

Der Schlauch mit den beiden Folienlagen 29 und 30 wird dann zu Beuteln 10 verarbeitet in der Weise, daß zunächst die Schweißungen 11, 12 sowie 13, 18 wie 19, 20 sowie 21, 22 angebracht werden und somit die beiden Verbundfolienlagen miteinander verbunden werden. Dadurch ist der Beutel geschlossen. Es können auch noch, wie in Figur 6 dargestellt, die Kanäle 23 und 24 nach Figur 1 durch eine an deren vorderen Eingang 33 vorhandene Schweißung geschlossen werden. In der Regel ist dies jedoch nicht notwendig, weil die Folienlagen dicht einander anliegen. Nachdem somit durch die vorgenannten Schweißungen 11 bis 22 die Beutel geschlossen sind, dann werden die zu Figur 1 angegebenen Schnitte 25, 26, 27 und 28 angebracht und somit der Beutel besäumt. Dadurch wird zwar der Schlauch geöffnet, aber diese Schnitte sind ohne Einfluß auf das Innere des Beutels.

Figur 6 zeigt, daß ausgehend von einem entsprechend breiten Schlauch, nebeneinander jeweils zwei Beutel hergestellt werden.

Es sei bemerkt, daß die Besäumungsschnitte 25 bis 28 auch im unmittelbaren Anschluß an die Herstellung der Schweißnähte 13 bis 22 erfolgen können, d. h. falls für die Herstellung der Schweißnähte und der Schnitte ein Werkzeug vorhanden ist.

Zur Herstellung der Beutel ist besonders vorteilhaft, einen Ausgangsschlauch aus Polyäthylen (PE) oder Polypropylen (PP) durch weitere Folienlagen zu kaschieren, weil durch die Wahl dieser Folien im Hinblick auf chemische Zusammensetzung, Dichte und Dicke eine spezielle Anpassung an die im Beutel zu speichernde Flüssigkeit erfolgen kann. Dazu wird nach Figur 7 ebenfalls unter aseptischen Bedingungen der Innenschlauch 29 geblasen. Auf diesen wird dann zu beiden Seiten eine Folienlage 30, 30a aufkaschiert. Dieser Schlauch aus Verbundfolie wird im unmittelbaren Anschluß zu Beuteln verarbeitet oder zu einer Rolle 34 aufgewickelt, die später, wie die nachfolgenden Figuren zeigen, zu den Beuteln für Infusionslösungen verarbeitet wird.

Figur 8 zeigt, daß die Rolle 34 mit Beuteln versehen wird, deren Kanäle 23, 24 zur Längskante 35 des Schlauches quer verlaufen und somit zu dieser gerichtet sind. In Figur 8 sind die Beutel einfacher gestaltet als in Figur 1. So fehlt auch die im Bereich der Anschlußteile doppelte, in einem Abstand zueinander befindliche Schweißung. Vielmehr ist lediglich eine Schweißung 18 vorhanden. Auch am Bodenende ist lediglich eine das Beutelvolumen bestimmende Schweißung 14 vorhanden. Figur 8 zeigt weiterhin, daß außen neben den vorerwähnten Schweißungen 18, 25, 26 sowie 14 eine Perforation 36 vorhanden ist, die neben den Schweißnähten zur Verbindung der beiden Folienlagen angeordnet ringsumlaufend ist, damit der Schlauch aus Verbundfolienmaterial eine Beutelkette bildet und die Beutel erst dann vom Schlauch getrennt werden, sofern sie mit der Flüssigkeit befüllt sind.

Um die Beutel später bei ihrer Anwendung aufhängen zu können, ist die Perforationslinie 36a in einem größeren Abstand zu der Bodenschweißung 14 vorhanden. In dem dazwischen verbleibenden Raum sind Aufhängelöcher eingestanzt, und zwar vorteilhaft durch mehrere, an einem Träger 37 angeordnete erhitzte Dorne 38, 38a usw. Die erhitzten Dorne werden vorgeschlagen, damit zugleich mit den Lochungen die Schnittränder miteinander verschweißt werden. Dadurch erfolgt kein « Öffnen » des Schlauches. Diese durch die Dorne 38 vorgenommenen Lochungen 39, 39a usw. haben vorteilhaft einen solchen Abstand zueinander, der jeweils gleich ist, und zwar über die gesamte Länge des Schlauches. Dadurch ist es möglich, diese Lochungen auch für weitere Zwecke, hier z. B. dem Transport des Schlauches mit Beuteln zu verwenden, beispielsweise zu oder in einer Füllstation zum Befüllen der Beutel, wie das noch nachfolgend beschrieben werden wird.

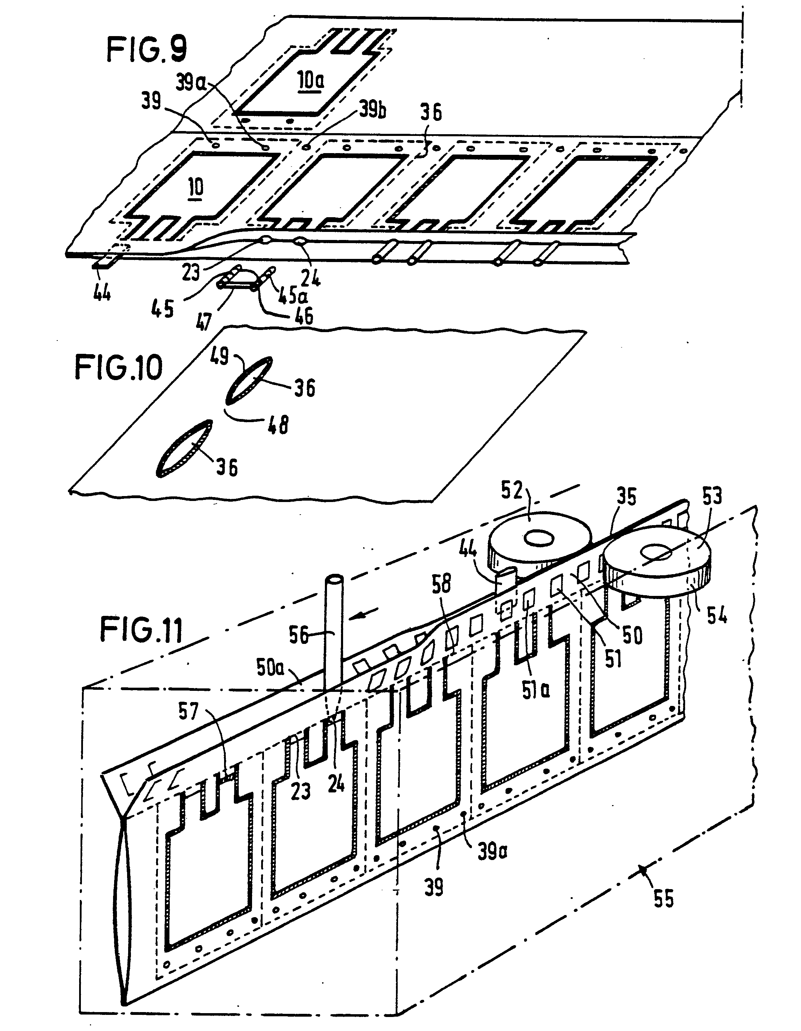

Nach Figur 8 haben die vorderen Enden der Kanäle 23 und 24 zu der Schlauchlängskante 35 einen Abstand, dessen Vorteile noch in Verbindung mit den Figuren 9 und 10 beschrieben werden.

Um die Folienlagen der Anschlußteile die die später Einfüll- oder Entleerungskanäle 23, 24 darstellen, in eine solche Haftung oder Anlage zueinander zu bringen, daß keine Kontaminierung dieser Kanäle stattfinden kann, sind in Figur 7 im Anschluß an die Extrusion aus den Düsen 40 des Extruders 41 Walzen 42 und 43 vorhanden, die nur von kurzer wirksamer Länge sind und lediglich in dem Schlauch 29 einen Streifen bilden, in dem die einander zugekehrten Innenflächen des Schlauches im Bereich der Kanäle 23, 24 zu einer gewissen Anhaftung gelangen. Der Anlagedruck der Walzen 42 und 43 ist einstellbar, so daß damit auch die Kraft, mit der die Folienlagen im Bereich der Kanäle 23 und 24 anliegen, einstellbar ist.

Figur 9 zeigt, daß in einem breiten Schlauch spiegelbildlich gegenüberliegend jeweils gleichzeitig zwei Beutel 10, 10a hergestellt werden. Auch zeigt Figur 8, daß die Lochungen 39, 39a einen solchen gleichbleibenden Abstand haben, daß gegebenenfalls auch Lochungen 39b im Schlauch angeordnet und nicht lediglich in einem durch Perforation 36 umgrenzten Beutel zugeordnet sind.

Nach Figur 9 wird mittels eines Messers 44 die Schlauchlängskante 35 aufgeschnitten. Dadurch werden die Einlaßöffnungen der Kanäle 23 und 24 freigelegt. Dies bedeutet, daß die Kanäle 23 und 24 unabhängig davon, ob deren Wandungen mit einer gewissen Haftung einander anliegen und somit ohnehin dicht sind, daß erst zu einem gewünschten Zeitpunkt die Schlauchlängskante aufgeschnitten und dadurch eine Zugänglichkeit zu den Kanälen gegeben wird. Nach Figur 9 werden die Kanäle mit Stopfen 45 und 45a versehen, die ringsumlaufen Vorsprünge 46 haben, um deren Haftung zu erhöhen und Widerhaken darstellen, damit diese Stopfen nicht aus den Öffnungen herausgelangen können. Die Stopfen 45 und 46a sind mit bei deren Herstellung aus spritzgegossenem Kunststoff angebrachten Verbindungsschnüren 47 versehen. Da die Öffnungen der Kanäle 23 und 24 der Beutel in einer Reihe liegen und gleichmäßige oder jeweils von Beutel zu Beutel gleichmäßige Abstände haben, ist es einfach möglich, die Stopfen 45, 45a oder dergleichen Elemente maschinell in die Kanäle 23 und 24 einzufügen.

Figur 10 zeigt in größerem Detail Perforationsschnitte 36, zwischen denen ein Verbindungssteg 48 vorhanden ist. Die Perforationsschnitte 36 werden hergestellt durch erhitzte, mit Zacken versehene Messer mit dem Ergebnis, daß die Schnittkanten durch Schweißungen 49 miteinander verbunden sind. Dadurch ergibt sich ebenfalls, daß kein « Öffnen » des Schlauches stattfindet, sondern auch die Schlauchbereiche zwischen den Beuteln hermetisch abgeschlossen bleiben.

Figur 11 zeigt, daß der Randbereich 50 und 50a zwischen der Längskante 35 und den vorderen Enden der Anschlußteile bzw. Kanäle 23, 24 verschiedene Funktionen ausübt. So ist dieser Randbereich versehen mit fensterartigen Öffnungen 51 und 51a, die vorzugsweise rechteckig sind nach Art der Ausstanzungen in Filmen für fotografische Zwecke. Vorteilhaft sind auch diese Ausstanzungen 51 und 51a an ihren Schnittlinien durch Anwendung eines erhitzten Stanzwerkzeuges verschweißt, so daß auch hier keine « Öffnung » des Schlauches stattfindet. Diese Ausnehmungen dienen dem Transport und auch der Steuerung der Befülleinrichtung.

Es brauchen aber nicht solche Ausnehmungen 51 oder dergleichen vorhanden zu sein, weil auch möglich ist, die Beutelkette durch Förderelemente, wie beispielsweise gegenüberliegende Räder 52 und 53 zu transportieren, deren dem Schlauch zugerichtete Seiten 54 mit rauher Oberfläche ausgebildet sind, um den Schlauch sicher transportieren zu können. Diese rauhe Oberfläche ist möglich, weil der Randstreifen 50 auch leicht beschädigt werden könnte, da er ohnehin später nach dem Befüllen der Beutel abgetrennt wird.

Mit 55 ist allgemein und in gestrichelter Linie eine Befüllstation dargestellt, die nach außen abgeschlossen ist und unter aseptischen Bedingungen tätig ist. Innerhalb dieser Füllstation 55 wird der Schlauch an seiner längsverlaufenden Kante 35 durch das Messer 44 aufgeschnitten, so daß erst in dieser Befüllstation die Öffnungen der Kanäle 23 und 24 frei liegen. In die jeweiligen Einfüllöffnungen (Kanal 24) wird das vorne angespitzte Befüllröhrchen 56 eingeführt, wodurch die Lösung in den Beutel gelangt. Das Einführen wird vereinfacht durch die V-förmige Wandung der Randstreifen 50 und 50a. Durch das Einführen des Füllröhrchens 56 werden die gegebenenfalls stärker einander anliegenden Wandungen des Kanals 24 einander entfernt, so daß das Innere des Beutels erst innerhalb der unter aseptischen Bedingungen arbeitenden Befüllkammer zugänglich ist.

Nachdem die Beutel gefüllt worden sind, werden gegebenenfalls die zu Figur 9 beschriebenen Stopfen 45 und 45a eingebracht. Nach dem diese in die zugeordnete Öffnung der Kanäle 23 oder 24 eingebracht sind, können sie zusätzlich zu den Vorsprüngen 46 noch durch eine ringsumlaufende Verschweißung gesichert werden. Es ist jedoch auch möglich, einen befüllten Beutel an seinem einstückigen Anschlußteil mit einer Verschlußschweißung 57 zu versehen, wie das in Figur 11 dargestellt ist.

Sobald die Beutel mit der Flüssigkeit gefüllt werden, werden sie von der flachen zu der dreidimensionalen Raumform übergehen. Dabei werden die zu Figur 10 beschriebenen Stege 48 getrennt, so daß die Beutel vom Schlauch befreit werden. Es ist jedoch auch möglich, daß die Trennung der Beutel zu einem späteren Zeitpunkt erfolgt. Vorteilhaft ist aber in jedem Fall, den oberen Randstreifen 50 entlang der Perforationslinie 58 abzutrennen.

Die erfindungsgemäße Lösung ergibt durch die Maßgabe, daß die Einfüllöffnungen erst in einer Befüllkammer frei liegen, eine sichere Gewähr, daß das Innere des Beutels, das steril hergestellt worden ist, auch beim Befüllen steril erhalten worden ist. Dadurch können die Beutel sicher bis zu ihrer Befüllung gelagert und auch transportiert werden.

Obwohl die Beutel steril hergestellt und auch in diesem Zustand ihre Befüllungen erhalten, kann es zweckmäßig sein, nach der Befüllung oder während der Befüllung in der Befüllkammer 55 eine Sterilisation bei einer Temperatur von etwa 120° und unter Anwendung von Vakuum oder eine Sterilisation mit Gamma-Strahlen durchzuführen.

**Patentansprüche**

1. Beutel für Infusionslösungen oder dergleichen, der ausgehend von einem unter aseptischen Bedingungen hergestellten Schlauch aus thermoplastischer Kunststoffolie durch Verschweißung der beiden Schlauchwandungen (29, 30) und Trennschnitte (25, 26, 27, 28) durch die Schlauchwandungen hergestellt und mit einem nach außen vorstehenden, durch Stopfen (45, 45a) verschließbaren Anschlußteil oder mehreren solcher Anschlußteile (23, 24) versehen ist, dadurch gekennzeichnet, daß die beiden gegenüberliegenden Schlauchwandungen jeweils aus einer Verbundfolie (29, 30) bestehen, die Verbundfolien durch die Umfangskontur des Beutels (10, 10a) nachzeichnenden Schweißungen (13, 14, 17, 18) miteinander verbunden und außen neben diesen Schweißungen die Trennschnitte (25, 26, 27, 28) vorhanden sind sowie das Anschlußteil oder die Anschlußteile (23, 24) mit dem Beutel (10, 10a) einstückig ist bzw. sind.

2. Beutel nach Anspruch 1, dadurch gekennzeichnet, daß das Anschlußteil oder die Anschlußteile (23, 24) zur Schlauchlängskante (35) einen Abstand haben.

3. Beutel nach Anspruch 1, dadurch gekennzeichnet, daß die nach außen vorstehenden Anschlußteile (23, 24) in an sich bekannter Weise konisch sind und Kanäle bilden, deren Querschnitte sich in Richtung von außen nach innen verjüngen.

4. Beutel nach Anspruch 1, dadurch gekennzeichnet, daß er in seinem Bodenbereich und in seinem Kopfbereich mit zwei im Abstand zueinander befindlichen Querabschweißungen (13, 14 und 17, 18) versehen ist.

5. Schlauch mit Beuteln nach Anspruch 2, dadurch gekennzeichnet, daß zwischen den einzelnen durch Verschweißung der Folienlagen (29, 30) des Schlauches gebildeten Beuteln (10, 10a) durch beide Schlauchwandungen hindurchgehende Perforationsschnitte (36) angeordnet sind.

6. Schlauch nach Anspruch 5, dadurch gekennzeichnet, daß die Perforationsschnitte (36) durch erhitzte mit Zacken (38) versehene Messer (37) erhalten und dabei die Schnittflächen der Folienlagen (29 30) durch Schweißung (49) miteinander verbunden sind.

7. Schlauch nach Anspruch 2, dadurch gekennzeichnet, daß der von den Anschlußteilen (23, 24) freie Schlauchlängskantenbereich (50) mit dem Transport des Schlauches dienenden Elementen (51, 51a) versehen ist.

8. Schlauch nach Anspruch 7, dadurch gekennzeichnet, daß der von den Anschlußteilen (23, 24) freie Schlauchlängskantenbereich (50) mit hintereinander angeordneten Ausschnitten (51, 51a) versehen ist.

9. Schlauch nach Anspruch 8, dadurch gekennzeichnet, daß die Ausschnitte (51, 51a) durch beide Schlauchwandungen (29) wie bei Filmen zum Herstellen von Fotografien ausgebildet sind.

10. Schlauch nach Anspruch 5, dadurch gekennzeichnet, daß im Bereich der den Anschlußteilen (23, 24) der Beutel (10, 10a) abgekehrten Kante des Schlauches außerhalb der Beutel durch beide Schlauchwandungen durchgehende Durchbrechungen (39, 39a) vorhanden sind.

11. Schlauch nach Anspruch 10, dadurch gekennzeichnet, daß die Durchbrechungen (39, 39a) den

**0 096 191**

einzelnen Beuteln (10, 10a) zugeordnet sind.

12. Schlauch nach Anspruch 5, dadurch gekennzeichnet, daß die den Anschlußteilen (23, 24) zugekehrte Schlauchlängskante (35) aufgeschnitten ist.

13. Beutel nach Anspruch 1, dadurch gekennzeichnet, daß die innere Folienlage (29) aus einem Folienmaterial mit niedrigem Schmelzpunkt und die äußere Folienlage (30) aus einem Folienmaterial mit höherem Schmelzpunkt besteht.

14. Beutel nach den Ansprüchen 1 und 2, dadurch gekennzeichnet, daß in an sich bekannter Weise jede Wandung des Beutels (10, 10a) aus mehr als zwei Folienlagen besteht.

15. Beutel nach Anspruch 13, dadurch gekennzeichnet, daß in an sich bekannter Weise die innere Folienlage (29) aus Polyäthylen (PE) und die äußere Folienlage (30) aus einem Polyamid (PA) besteht.

16. Beutel nach Anspruch 1, dadurch gekennzeichnet, daß die innere Folienlage (29) aus Polypropylen (PP) und die äußere Folienlage (30) aus Polyamid (PA) besteht.

17. Beutel nach Anspruch 14, dadurch gekennzeichnet, daß zwischen der Außenfolie aus Polyamid (PA) und der Innenfolie aus Polyäthylen (PE) oder Polypropylen (PP) eine weitere aus anderem thermoplastischem Kunststoff bestehende Folienlage vorhanden ist.

18. Beutel nach Anspruch 17, dadurch gekennzeichnet, daß die mittlere Folienlage aus einem orientierten Polyester mit X-Beschichtung (OPX) besteht.

19. Beutel nach Anspruch 16, dadurch gekennzeichnet, daß in Richtung von außen nach innen vorhanden sind

a) Außenlage aus Polyamid (PA)
b) innere Lage aus Polyäthylen (PE)
c) innere Lage aus Polyvinylidenchlorid (PVDC)
d) Innenlage aus Polyäthylen (PE)

20. Verfahren zum Herstellen der Beutel nach Anspruch 1, dadurch gekennzeichnet, daß zunächst unter aseptischen Bedingungen ein Schlauch (29) aus thermoplastischer Kunststoffolie, insbesondere aus Polyäthylen, hergestellt und an diesen Schlauch zu beiden Seiten jeweils eine Folienlage (30, 30a) aus anderem thermoplastischem Kunststoff, insbesondere aus einem Polyamid, durch Kaschierung angebracht und aus diesem Schlauch die Beutel (10, 10a) in der Weise hergestellt werden, daß die so erhaltenen beiden Verbundfolien der Umfangskontur der Beutel entsprechend durch Schweißungen (13, 14, 17, 18) miteinander verbunden werden und außen neben diesen Schweißungen Trennschnitte (25, 26, 27, 28) angebracht werden.

21. Verfahren nach Anspruch 20, dadurch gekennzeichnet, daß der Schlauch (29) bei einer Extrusionstemperatur von ca. 170 °C hergestellt wird und an dem hermetisch geschlossenen Schlauch die Verschweißung der beiden Schlauchwandungen zur Herstellung der Beutel (10, 10a) vorgenommen und außen neben den Schweißnähten (13, 14, 17, 18) der Beutel besäumt wird.

22. Verfahren nach Anspruch 20, dadurch gekennzeichnet, daß in dem Schlauch (29) in dichtem Abstand hintereinander und quer zur Längserstreckung des Schlauches mit zu einer Schlauchlängskante (35) gerichteten Anschlußteilen (23, 24) und in einem Abstand zur Schlauchlängskante durch Verschweißung der beiden Folienlagen (29, 30) die Beutel (10, 10a) hergestellt und zwischen den einzelnen Beuteln in einer Erstreckung quer zur Beutellängsrichtung durch beide Schlauchwandungen durchgehend Perforationsschnitte (36) angebracht werden und in einer unter aseptischen Bedingungen arbeitenden Füllstation der Schlauch an seiner Längskante aufgeschnitten und dadurch die Anschlußteile (23, 24) freigelegt und die Beutel befüllt sowie anschließend die Öffnungen der Anschlußteile geschlossen werden.

23. Verfahren nach Anspruch 22, dadurch gekennzeichnet, daß die Öffnungen unter aseptischen Bedingungen durch Stopfen (45, 45a) verschlossen werden.

## Claims

1. Sachet, for infusion solutions or the like, which starting out from a hose produced under aseptic conditions from thermoplastic synthetic material film is produced through welding both the hose walls (29, 30) together and severing cuts (25, 26, 27, 28) through the hose walls and provided with an outwardly protruding connecting part closable by plugs (45, 45a) or several such connecting parts (23, 24), characterised thereby, that both the oppositely disposed hose walls each consist of a respective compound film (29, 30), the compound films are connected together by welds (13, 14, 17, 18) tracing the peripheral outlines of the sachet (10, 10a) and the severing cuts (25, 26, 27, 28) are present outside beside these welds as well as that the connecting part or parts (23, 24) is or are integral with the sachet (10, 10a).

2. Sachet according to claim 1, characterised thereby, that the connecting part or parts (23, 24) has or have a spacing from the longitudinal edge (35) of the hose.

3. Sachet according to claim 1, characterised thereby, that the outwardly protruding connecting parts (23, 24) in an itself known manner are conical and form channels, the cross-sections of which narrow in direction from outside to inside.

4. Sachet according to claim 1, characterised thereby, that it is provided in its base region and in its head region with two transverse welds (13, 14 and 17, 18) disposed at a spacing one from the other.

9

5. Hose with sachets according to claim 2, characterised thereby, that perforating cuts (36) passing through both hose walls are arranged between the individual sachets (10, 10a) formed through welding together of the film layers (29, 30) of the hose.

6. Hose according to claim 5, characterised thereby, that the perforating cuts (36) are obtained through heated blades (37) provided with prongs (38) and the cut faces of the film layers (29, 30) are in that case connected together through welding (49).

7. Hose according to claim 2, characterised thereby, that that longitudinal edge region (50) of the hose, which is free of the connecting parts (23, 24), is provided with elements (51, 51a) serving for the transport of the hose.

8. Hose according to claim 7, characterised thereby, that that longitudinal edge region (50) of the hose, which is free of the connecting parts (23, 24), is provided with cut-outs (51, 51a) arranged one behind the other.

9. Hose according to claim 8, characterised thereby, that the cut-outs (51, 51a) are formed through both hose walls (29) as in films for the production of photographs.

10. Hose according to claim 5, characterised thereby, that passages (39, 39a) passing through both hose walls externally of the sachets are present in the region of that edge of the hose, which is remote from the connecting parts (23, 24) of the sachets (10, 10a).

11. Hose according to claim 10, characterised thereby, that the passages (39, 39a) are associated with the individual sachets (10, 10a).

12. Hose according to claim 5, characterised thereby, that longitudinal edge (35) of the hose, which faces the connecting parts (23, 24), is cut open.

13. Sachet according to claim 1, characterised thereby, that the inner film layer (29) consists of a film material of low melting point and the outer film layer (30) consists of a film material of higher melting point.

14. Sachet according to the claims 1 and 2, characterised thereby, that each wall of the sachet (10, 10a) in an itself known manner consists of more than two film layers.

15. Sachet according to claim 13, characterised thereby, that in an itself known manner, the inner film layer (29) consists of polyethylene (PE) and the outer film layer (30) consists of a polyamide (PA).

16. Sachet according to claim 1, characterised thereby, that the inner film layer (29) consists of polypropylene (PP) and the outer film layer (30) consists of polyamide (PA).

17. Sachet according to claim 14, characterised thereby, that a further film layer consisting of different thermoplastic synthetic material is present between the outer film of polyamide (PA) and the inner film of polyethylene (PE) or polypropylene (PP).

18. Sachet according to claim 17, characterised thereby, that the middle film layer consists of an oriented polyester with X-coating (OPX).

19. Sachet according to claim 16, characterised thereby, that

a) outer layer of polyamide (PA)
b) inner layer of polyethylene (PE)
c) inner layer of polyvinylidenchloride (PVDC)
d) inside layer of polyethylene (PE)

are present in direction from outside to inside.

20. Method for the production of the sachets according to claim 1, characterised thereby, that initially a hose (29) is produced under aseptic conditions from thermoplastics synthetic material film, in particular from polyethylene, and a respective film layer (30, 30a) of different thermoplastic synthetic material, in particular of a polyamide, is applied through laminating at this hose to both sides thereof and the sachets (10, 10a) are produced from this hose in the manner that both the compound films thus obtained are connected together by welds (13, 14, 17, 18) in correspondence with the peripheral outline of the sachets and severing cuts (25, 26, 27, 28) are applied outside beside these welds.

21. Method according to claim 20, characterised thereby, that the house (29) is produced at an extrusion temperature of about 170 ºC and the welding together of both the hose walls of the production of the sachets (10, 10a) is undertaken at the hermetically closed hose and the sachet is trimmed outside beside the welding seams (13, 14, 17, 18).

22. Method according to claim 20, characterised thereby, that the sachets (10, 10a) are produced through welding together of both the film layers (29, 30) in the hose (29) at close spacing one behind the other and transversely to the longitudinal extent of the hose with connecting parts (23, 24) directed towards a longitudinal edge (35) of the hose and at a spacing from the longitudinal edge of the hose and perforating cuts (36) passing through both hose walls are applied between the individual sachets in an extent transverse to the longitudinal direction of the sachets and the hose is cut open at its longitudinal edge in a filling station operating under aseptic conditions and the connecting parts (23, 24) are thereby exposed and the sachets are filled as well as the openings of the connecting parts closed subsequently.

23. Method according to claim 22, characterised thereby, that the openings are closed under aseptic conditions by plugs (45, 45a).

**Revendications**

1. Poche pour solution de perfusions ou autres, fabriquée à partir d'une gaine en film thermoplastique obtenu dans des conditions aseptiques, par soudage des deux parois de gaine (29, 30) et des perforations (25, 26, 27, 28) pratiquées à travers les parois de la gaine, la poche étant munie d'une ou de plusieurs pièces de raccordement (23, 24) faisant saillie vers l'extérieur, pouvant être obturées par des bouchons (45, 45a), poche caractérisée en ce que les deux parois opposées de la gaine sont constituées chacune par un film composite (29, 30), que les films composites sont reliés entre eux par les soudures (13, 14, 17, 18) du contour périphérique de la poche (10, 10a) et qu'en plus de ces soudures les perforations (25, 26, 27, 28) sont présentes et que la ou les pièces de raccordement (23, 24) sont formées intégralement avec la poche (10, 10a).

2. Poche selon la revendication 1, caractérisée en ce que la ou les pièces de raccordement (23, 24) sont situées à une certaine distance de l'arête longitudinale de la gaine (35).

3. Poche selon la revendication 1, caractérisée en ce que les pièces de raccordement faisant saillie vers l'extérieur (23, 24) présentent une forme conique connue en soi et constituent des canaux dont la section se réduit de l'extérieur vers l'intérieur.

4. Poche selon la revendication 1, caractérisée en ce qu'elle est munie, dans sa zone inférieure et dans sa zone de tête, de deux soudures transversales séparées l'une de l'autre (13, 14 et 17, 18).

5. Gaine avec poche selon la revendication 2 caractérisée en ce qu'entre les poches (10, 10a) individuelles, formées par soudage des couches de film (29, 30) de la gaine, des perforations (36) traversant les deux parois de la gaine sont prévues.

6. Gaine selon la revendication 5, caractérisée en ce que les perforations (36) sont obtenues à l'aide d'une lame (37) munie de dents (38) et qu'ainsi les surfaces de coupe des couches de film (29, 30) sont reliées entre elles par la soudure (49).

7. Gaine selon la revendication 2, caractérisée en ce que la zone de bordure (50) de la gaine exempte des pièces de raccordement (23, 24) est munie d'éléments (51, 51a) servant au transport de la gaine.

8. Gaine selon la revendication 7, caractérisée en ce que la zone (50) de l'arête longitudinale de la gaine exempte des pièces de raccordement (23, 24) est munie de découpes (51, 51a) disposées en alignement.

9. Gaine selon la revendication 8, caractérisée en ce que les découpes (51, 51a) sont constituées à travers les deux parois de gaine (29) de la même façon que sur les pellicules photographiques.

10. Gaine selon la revendication 5, caractérisée en ce qu'on trouve au voisinage de l'arête de gaine qui est éloignée des pièces de raccordement (23, 24) de la poche (10, 10a), des perforations (39, 39a) traversant les deux parois de gaine se trouvent à l'extérieur de la poche.

11. Gaine selon la revendication 10, caractérisée en ce que les perçages (39, 39a) se rattachent aux poches individuelles (10, 10a).

12. Gaine selon la revendication 5, caractérisée en ce que l'arête longitudinale (35) de la gaine tournée vers les pièces de raccordement (23, 24) est découpée.

13. Poche selon la revendication 1, caractérisée en ce que la couche de film intérieure (29) est constituée par un matériau de films ayant un faible point de fusion et que la couche de film extérieure (30) est constituée par un matériau de films ayant un point de fusion élevé.

14. Poche selon les revendications 1 et 2, caractérisée en ce que chaque paroi de la poche (10, 10a) est constituée par plus de deux couches de film, de manière connue en soi.

15. Poche selon la revendication 13, caractérisée en ce que d'une manière connue en soi, la couche de film intérieure (29) est en polyéthylène (PE) et que la couche de film extérieure (30) est en polyamide (PA).

16. Poche selon la revendication 1, caractérisée en ce que la couche de film intérieure (29) est en polypropylène (PP) et la couche de film extérieure (30) est en polyamide (PA).

17. Poche selon la revendication 14, caractérisée en ce qu'entre le film extérieur en polyamide (PA) et le film intérieur en polyéthylène (PE) ou en polypropylène (PP) se trouve une autre couche de film constituée par une autre matière thermoplastique.

18. Poche selon la revendication 17, caractérisée en ce que la couche centrale de film est constituée par un polyester orienté avec un revêtement X (OPX).

19. Poche selon la revendication 16, caractérisée en ce que, dans le sens de l'extérieur vers l'intérieur, on trouve

| a) une couche extérieure en polyamide | (PA) |
| b) une couche intérieure en polyéthylène | (PE) |
| c) une couche intérieure en chlorure de polyvinylidène | (PVDC) |
| d) une couche intérieure en polyéthylène | (PE) |

20. Procédé de fabrication d'une poche selon la revendication 1, caractérisé en ce que l'on fabrique d'abord une gaine (29) en film thermoplastique en particulier du polyéthylène, dans des conditions aseptiques, et que sur les deux faces de cette gaine on applique une couche de films (30, 30a) en matière thermoplastique, en particulier en polyamide, au moyen de collage-doublage et qu'à partir de cette gaine on fabrique les poches (10, 10a) de façon que les deux films composites ainsi obtenus soient reliés entre elles suivant le contour périphérique des poches par les soudures (13, 14, 17, 18) et qu'en plus des soudures des perforations (25, 26, 27, 28) soient présentes.

21. Procédé selon la revendication 20, caractérisé en ce que la gaine (29) est fabriquée à une température d'extrusion d'environ 170 °C et que sur la gaine hermétiquement fermée, le soudage des deux parois de gaine destiné à la production des poches (10, 10a) est effectué et qu'en plus des soudures (13, 14, 17, 18), les poches sont cisaillées.

22. Procédé selon la revendication 20, caractérisé en ce que les poches (10, 10a) sont produites dans la gaine (29) à faible distance les unes derrière les autres et perpendiculairement au sens longitudinal de la gaine avec des pièces de raccordement (23, 24) dirigées vers une arête longitudinale (39) de la gaine, et à une certaine distance de l'arête longitudinale de la gaine, par soudage des deux couches de feuilles (29, 30), et qu'entre les poches individuelles, suivant une direction perpendiculaire à la direction longitudinale des poches, des perforations (36) traversant les deux parois de gaine sont pratiquées et que dans un poste de remplissage fonctionnant dans des conditions aseptiques, la gaine est découpée suivant son arête longitudinale et qu'ainsi les pièces de raccordement (23, 24) sont libérées et les poches remplies ainsi qu'ensuite les orifices des pièces de raccordement sont fermés.

23. Procédé selon la revendication 22, caractérisé en ce que les orifices sont fermés par des bouchons (45, 45a) dans des conditions aseptiques.

FIG.1

FIG.2

FIG.3

FIG.4

FIG.5

FIG.6

0 096 191

FIG.7

FIG.8

2

FIG.9

FIG.10

FIG.11